# EUROPÄISCHE PATENTSCHRIFT

(11) **EP 2 982 325 B1**
(45) Veröffentlichungstag und Bekanntmachung des Hinweises auf die Patenterteilung: **18.03.2020**
(21) Anmeldenummer: 14179742.3
(22) Anmeldetag: 04.08.2014
(51) Int. Cl.: A61B 18/14

(54) **Verfahren zur Herstellung einer Branche und chirurgisches Instrument mit einem Branchen aufweisenden Werkzeug**
Method for producing a branch and surgical instrument with a tool having branches
Procédé de fabrication d'une branche et d'un instrument chirurgical doté d'un outil présentant une branche

(43) Veröffentlichungstag der Anmeldung: 10.02.2016
(73) Patentinhaber: Erbe Elektromedizin GmbH, 72072 Tübingen (DE)
(72) Erfinder: Mayer, Volker, 72072 Tübingen (DE); Brodbeck, Achim, 72555 Metzingen (DE)
(74) Vertreter: Rüger Abel Patentanwälte PartGmbB

(56) Entgegenhaltungen:
- EP-A1- 2 301 467
- EP-A1- 2 510 896
- EP-A1- 2 807 988
- US-A1- 2009 082 767

## Beschreibung

Die Erfindung betrifft ein Verfahren zur Herstellung einer Branche für ein chirurgisches Instrument sowie ein Instrument mit wenigstens einer durch das Verfahren bereitgestellten Branche.

Aus der EP 2 554 132 ist ein Instrument zur Koagulation von biologischem Gewebe zwischen zwei Branchen eines Werkzeugs eines chirurgischen Instruments bekannt. Jede Branche besteht aus einem Elektrodenträger, der über mehrere punktartige Schweißverbindungen mit einer dünnen plattenförmigen Elektrode verbunden ist. Der Elektrodenträger kann eine Kunststoffummantelung aufweisen.

Weiter ist aus der US 2011/0073246 A1 ein Instrument bekannt, dessen Branchen aus einem Kunststoff-Metall-Verbundbauteil bestehen. Dieses wird durch eine Elektrodenplatte mit abgewinkeltem Rand gebildet, der Randvertiefungen oder Löcher aufweist. Die Elektrodenplatte wird mit Kunststoff unterspritzt, der sich über den Rand der Elektrodenplatte erstreckt.

Auch die WO 02/080785 A1 befasst sich mit der Verankerung von Elektrodenplatten in einem elektrisch isolierenden Substrat bei Branchen eines medizinischen Instruments. Die Elektrodenplatte kann einen gekröpften Rand aufweisen, der mit dem Kunststoffsubstrat in formschlüssigem Eingriff steht.

Weiter ist aus der EP 2 807 988 A1 ein Verfahren zur Herstellung einer Branche bekannt, die aus Kunststoff und Metalleinlagen besteht. Diese werden zunächst als zusammenhängendes Stanzteil bereitgestellt und mit teilweise frei liegenden Stegen mit Kunststoff umspritzt. Die frei liegenden Teile werden später abgetrennt.

Bei der Konzeption medizinischer Instrumente zum Koagulieren von Gewebe ist zu beachten, dass das zangenartige Werkzeug präzise ausgebildet sein und relativ hohen Druck auf biologisches Gewebe ausüben können muss. Weitere Restriktionen wie Keimfreiheit, Sterilisierbarkeit, Wärmebeständigkeit, elektrische Isolierung der Elektrodenplatten, thermische Isolierung der Elektrodenplatten zum umgebenden Gewebe, usw. treten hinzu.

Davon ausgehend ist es Aufgabe der Erfindung, ein Konzept anzugeben, mit dem sich verbesserte Branchen zur Herstellung qualitativ hochwertiger medizinischer Instrumente erzeugen lassen.

Diese Aufgabe wird mit dem Verfahren nach Anspruch 1 und dem elektrochirurgischen Instrument nach Anspruch 5 gelöst:
Bei dem erfindungsgemäßen Verfahren wird zunächst ein Metallteil bereitgestellt, das einen Trägerabschnitt und einen Funktionsabschnitt aufweist, die an der fertigen Branche elektrisch voneinander isoliert sein sollen, zunächst jedoch über einen elektrisch leitenden Verbindungssteg miteinander einteilig verbunden sind. Dieses Metallteil wird mit Kunststoff umspritzt, wobei wenigstens ein Oberflächenbereich des Funktionsabschnitts freigelassen werden kann, wie es vorzugsweise der Fall ist. Vorzugsweise wird auch der Verbindungssteg wenigstens teilweise freigelassen. Der Kunststoff stellt eine mechanische Verbindung zwischen dem Trägerabschnitt und dem Funktionsabschnitt her, d.h. er berührt beide Abschnitte und bildet somit eine stoffschlüssige und/oder formschlüssige Verbindung. Ist diese hergestellt, wird der Verbindungssteg entfernt. Dies kann, falls der Verbindungssteg mit umspritzt wurde, auch unter teilweiser Entfernung des Kunststoffs erfolgen. Wurde der Verbindungssteg freigelassen, kann er entfernt werden, ohne die Oberfläche des Kunststoffs zu durchdringen. Die Entfernung des Verbindungsstegs kann durch Abschneiden, Abbrechen, Abschleifen, Lasertrennen oder jedes andere geeignete Trennverfahren erfolgen.

Nach der Entfernung des Verbindungsstegs bzw. der Verbindungsstege sind der Trägerabschnitt und der Funktionsabschnitt voneinander elektrisch getrennt. Sie bleiben jedoch durch Vermittlung des Kunststoffs fest miteinander verbunden und bilden somit ein weitgehend spaltfreies, kompaktes Bauteil. Durch die fehlende metallische Verbindung zwischen Funktionsabschnitte, Trägerabschnitte sind diese nicht nur elektrisch gegeneinander isoliert, sondern es ist auch der Wärmeübergang zwischen dem Funktionsabschnitt und dem Trägerabschnitt vermindert.

Bei einer vorteilhaften Ausführungsform wird das Metallteil in einem Urformverfahren, beispielsweise in einem additiven Fertigungsverfahren bereitgestellt, beispielsweise indem zum Aufbau des Metallteils zu einem bestehenden Metallteil flüssige Metalltröpfchen lokal hinzugefügt werden. Alternativ kann das Metallteil in einem Gussverfahren, beispielsweise einem Feingussverfahren oder dem Metallspritzguss (MIM - Metal Injection Molding), hergestellt werden. Bei einer bevorzugten Ausführungsform wird das Metallteil durch ein pulvermetallurgisches Fertigungsverfahren bereitgestellt, beispielsweise durch selektives Laserschmelzen von Metallpulver. Dabei wird das Metallteil Schicht für Schicht durch Laserschmelzen in einem Bett aus Metallpulver erzeugt. Es können dabei komplizierte Geometrien mit vielfachen Hinterschneidungen, engen Spalten und dergleichen, erzeugt werden. Andere 3D-Druckverfahren zur Herstellung von Metallteilen können ebenfalls Anwendung finden.

Der Funktionsabschnitt der Branche kann eine Elektrodenplatte, ein Hebelarm oder ein sonstiger Abschnitt sein, der von dem Trägerabschnitt elektrisch und/oder thermisch zu trennen, mechanisch bzw. formschlüssig und/oder stoffschlüssig mit diesem aber verbunden ist.

Der Funktionsabschnitt und der Trägerabschnitt werden vorzugsweise derart bereitgestellt, dass sie miteinander einen Spalt festlegen. Dieser Spalt wird vorzugsweise vollständig mit einem Werkstoff, beispielsweise einem Kunststoff, ausgefüllt. In dem Spalt können an dem Funktionsabschnitt und/oder an dem Trägerabschnitt Haltestrukturen ausgebildet sein, die sich in Richtung auf den jeweils anderen Abschnitt erstrecken, diesen jedoch nicht berühren. Die Haltestrukturen sind vorzugsweise hinterschnittene Strukturen, wie beispielsweise Hakenformen, T-Strukturen, Pilzkopfstrukturen, Hammerkopfstrukturen oder dergleichen. Damit kann insbesondere derjenige Abschnitt, der einen freiliegenden Flächenbereich aufweist, insbesondere der Funktionsabschnitt, formschlüssig in dem Kunststoff verankert werden. Der Trägerabschnitt kann ebensolche Strukturen aufweisen oder vom Kunststoff ganz umschlossen sein, wodurch sich wiederum eine formschlüssige Verankerung des Kunststoffs an dem Trägerabschnitt ergibt. Der Werkstoff, mit dem der Funktionsabschnitt und der Trägerabschnitt verbunden werden, kann Bestandteile aufweisen, die eine stoffschlüssige Verbindung zwischen Funktionsabschnitt und Trägerabschnitt ermöglichen. So können der Funktionsabschnitt und der Trägerabschnitt formschlüssig und/oder stoffschlüssig miteinander verbunden werden.

Das erfindungsgemäße elektrochirurgische Instrument weist mindestens eine Branche auf, die einen Trägerabschnitt und einen Funktionsabschnitt aufweisen, die aus Metall gleicher Zusammensetzung und Struktur ausgebildet und über einen Körper, vorzugsweise einen Kunststoffkörper, miteinander fest verbunden sind. Die gleiche Zusammensetzung und Struktur des Metalls rührt vorzugsweise daher, dass der Trägerabschnitt und der Funktionsabschnitt gemeinsam in einem einzigen Urformvorgang bereitgestellt worden sind, wobei bei der Herstellung des Trägerabschnitts und des Funktionsabschnitts Verbindungsstege vorgesehen worden sind, die nach dem Ausfüllen des Spalts und/oder Umspritzen des Metallteils mit dem Verbindungswerkstoff beispielsweise dem Kunststoff entfernt worden sind. Der Trägerabschnitt und der Funktionsabschnitt sind somit Teile eines ehemals nahtlos einteiligen Bauteils.

Der Funktionsabschnitt, der eine Elektrodenplatte, ein Hebelarm oder dergleichen sein kann, ist mit dem Trägerabschnitt gemeinsam in einem Körper eingebettet und in diesem formschlüssig und/oder stoffschlüssig verankert. Dazu können Haltestrukturen vorgesehen sein, wie beispielsweise in den Spalt ragende Vorsprünge. Ein oder mehrere solcher Vorsprünge können an der dem Halteabschnitt zugewandten Seite des Funktionsabschnitts angeordnet sein. Zusätzlich oder alternativ können ein oder mehrere solcher Vorsprünge an der dem Funktionsabschnitt zugewandten Seite des Halteabschnitts angeordnet sein. Vorzugsweise weist zumindest einer der Vorsprünge eine Hinterschneidung auf. Dadurch ist eine sichere temperaturwechselstabile, belastbare Verbindung zwischen dem Funktionsabschnitt und dem Trägerabschnitt auch nach Entfernung der Verbindungsstege gegeben.

Weitere Einzelheiten vorteilhafter Ausführungsformen der Erfindung sind Gegenstand der Zeichnung, der Beschreibung oder von Ansprüchen. Es zeigen:
Figur 1 das erfindungsgemäße Instrument in ausschnittsweiser, perspektivischer Prinzipdarstellung,
Figur 2 eine Branche des Werkzeugs des Instruments nach Figur 1, in teilweise geschnittener vereinfachter Seitenansicht,
Figur 3 die Branche nach Figur 2, in Draufsicht,
Figur 4 die Branche nach Figur 2 und 3, in Querschnittsdarstellung,
Figur 5 ein Metallteil zur Ausbildung der Branche nach den Figuren 2 bis 4, in schematisierter Seitenansicht,
Figur 6 das Metallteil nach Figur 5 in größerer Darstellung, in geschnittener ausschnittsweiser Draufsicht und
Figur 7 das Metallteil nach Figur 5 und 6, in rückseitiger ausschnittsweiser Ansicht.

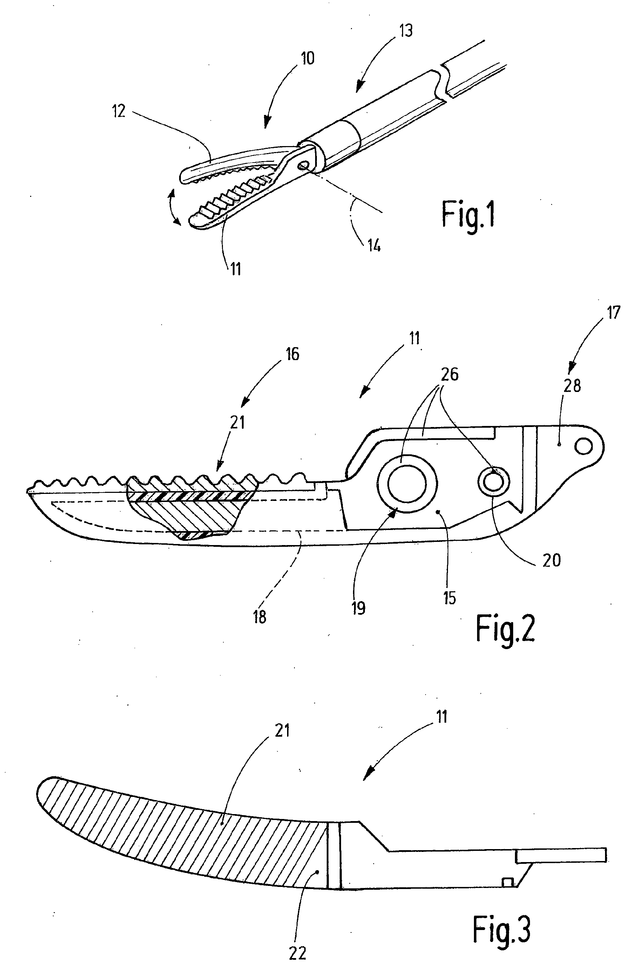

In Figur 1 ist der Werkzeugteil 10 eines chirurgischen Instruments veranschaulicht, das dazu dient, zwischen zwei Branchen 11, 12 des Werkzeugteils 10 gefasstes Gewebe zu koagulieren. Der Werkzeugteil 10 ist an einem Schaft 13 gehalten, durch den sich mindestens ein Betätigungselement für das Werkzeugteil 10 erstreckt, von dem mindestens eine der Branchen 11, 12 beweglich, insbesondere um eine Achse 14 schwenkbar ist.

Figur 2 veranschaulicht die Branche 11 exemplarisch. Die Branche 12 ist strukturell vorzugsweise ebenso aufgebaut, so dass die nachfolgende Beschreibung entsprechend für die Branche 12 gilt.

Die Branche 11 ist ein Kunststoff-Metall-Verbundbauteil. Zu ihm gehören ein Trägerabschnitt 15 und mindestens ein, im vorliegenden Falle zwei Funktionsabschnitte 16, 17. Der Trägerabschnitt 15, weist einen länglichen in Figur 2 gestrichelt veranschaulichten Finger 18 auf, der biegesteif ist, um die an der Branche 11 auftretenden Kräfte aufzunehmen. Außerdem kann in dem Trägerabschnitt 15 eine Scharnieröffnung 19 ebenso ausgebildet sein, wie weitere Strukturen, zum Beispiel in Form einer Durchgangsöffnung 20, zum Beispiel zur Befestigung weiterer Teile.

Der Funktionsabschnitt 16 wird im vorliegenden Ausführungsbeispiel durch eine Elektrodenplatte 21 gebildet, die mit dem zu koagulierenden biologischen Gewebe in Kontakt zu bringen ist. Die Elektrodenplatte 21 weist eine Funktionsfläche auf, die eben oder, wie in Figur 1 und 2 veranschaulicht, mit einer Struktur, beispielsweise in Form einer Reihe von Querrippen, versehen sein kann. Die Funktionsfläche liegt an der fertigen Branche 11 frei, so dass sie mit Gewebe in Berührung kommen kann. Die Elektrodenplatte 21 kann gerade oder wie in Figur 3 veranschaulicht, entlang ihrer Längsrichtung gekrümmt ausgebildet sein. Die Elektrodenplatte 21 ist vorzugsweise mit nicht weiter veranschaulichten Anschlussmitteln zur Anbringung einer elektrischen Zuleitung versehen. Diese kann im einfachsten Fall zum Beispiel an einer flachen Stelle 22 (Figur 3) der Elektrodenplatte 21 durch Punktschweißen befestigt sein. Abweichende Anschlussmöglichkeiten und Ausführungsformen sind denkbar. Es können auch Funktionsflächen vorgesehen werden, die von Kunststoff bedeckt sind.

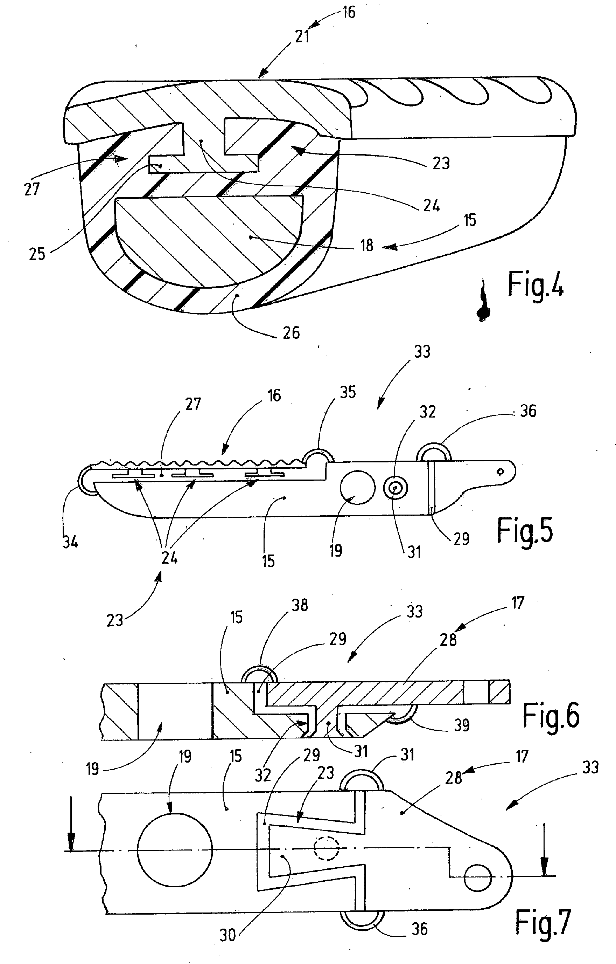

Der Funktionsabschnitt 16 kann mit einer Haltestruktur 23 versehen sein, die beispielsweise in Form eines oder mehrerer Fortsätze 24 ausgebildet ist. Solche Fortsätze 24 erstrecken sich vorzugsweise von der dem Trägerabschnitt 15 zugewandten Seite des Funktionsabschnitts 16 weg in Richtung auf den Trägerabschnitt 15 hin. Die Fortsätze 24 können kontinuierlich oder an einer oder mehreren Stellen in zunehmendem Abstand zu dem Funktionsabschnitt 16 einen vergrößerten Querschnitt aufweisen. In Figur 4 ist eine stufenartige Querschnittsvergrößerung veranschaulicht, wodurch der Fortsatz 24 einen Kopf 25 ausbildet. Dieser Kopf 25 bildet aus Sicht des Fingers 18 eine Hinterschneidung. Er dient zur Verankerung des Funktionsabschnitts 16 in einem Werkstoff, beispielsweise einem Werkstoffmantel, vorzugsweise einem Kunststoffmantel 26, mit dem der Trägerabschnitt 15 umspritzt ist. Der so gebildete Kunststoffmantel 26 füllt insbesondere einen zwischen dem Funktionsabschnitt 16 und dem Trägerabschnitt 15 ausgebildeten Spalt 27. Die sich in diesen Spalt 27 erstreckenden Fortsätze 24 verankern den Funktionsabschnitt 16 in dem Körper des Kunststoffmantels 26 formschlüssig. Der Kunststoffmantel 26 ist seinerseits formschlüssig an dem Trägerabschnitt 15 gehalten, indem er diesen umgreift. Der Trägerabschnitt 15 und der Funktionsabschnitt 16 sind thermisch und elektrisch voneinander getrennt. Eine metallische Brücke zwischen beiden besteht nicht.

Der weitere Funktionsabschnitt 17 der Branche 11 ist zum Beispiel ein Hebel 28, der mit dem Trägerabschnitt 15 mechanisch fest, jedoch ohne elektrischen Kontakt verbunden ist. Der Hebel 28 erstreckt sich von der Scharnieröffnung 19 aus gesehen in die dem Finger 18 entgegengesetzte Richtung. Der Hebel 28 dient zum Einleiten von Kräften in die Branche 11, um diese beispielsweise in Schließrichtung zu bewegen, um Gewebe zu fassen. Auch der Hebel 28 kann gegebenenfalls einen frei liegenden Flächenbereich, beispielsweise als Funktionsfläche, aufweisen, die nicht von Kunststoff umspritzt ist.

Der Aufbau des Funktionsabschnitts 17 ergibt sich insbesondere aus den Figuren 5 bis 7, die die Branche 11 vor Anbringung des Kunststoffmantels 26 veranschaulichen. Wiederum definieren der Funktionsabschnitt 17 und der Trägerabschnitt 15 miteinander einen Spalt 29, der im späteren Fertigungsverfahren von einem Werkstoff ausgefüllt wird. Der Spalt kann gerade oder abgewinkelt, gekröpft oder anderweitig geometrisch kompliziert geformt ausgebildet sein. Beispielsweise kann er, wie Figur 7 zeigt, eine schwalbenschwanzartige Kontur festlegen. Innerhalb dieser greift ein Fortsatz 30 des Funktionsabschnitts 17 bzw. des Hebels 28 in eine entsprechende Ausnehmung des Trägerabschnitt 15 um den Spalt 29 als Mäander festzulegen. Auch kann ein quer dazu gerichteter Fortsatz 31 des Hebels 28 in eine entsprechende Ausnehmung 32 des Trägerabschnitts 15 greifen. Der Spalt 29 kann sich somit um den zum Beispiel zylindrischen oder pilzförmigen Fortsatz 31 herum erstrecken.

Die Branche 11 wird wie folgt hergestellt:
Es wird zunächst ein Metallteil 33 bereitgestellt, wie es aus den Figuren 5 bis 7 ersichtlich ist. Dieses Metallteil 33 umfasst den Trägerabschnitt 15 sowie die Funktionsabschnitte 16, 17. Der Trägerabschnitt 15 ist mit dem Funktionsabschnitt 16 über mindestens einen, vorzugsweise mehrere Verbindungsstege 34, 35 verbunden. Ebenso ist der Funktionsabschnitt 17 mit dem Trägerabschnitt 15 über einen oder mehrere Verbindungsstege 36, 37, 38, 39 verbunden. Zur Herstellung des Metallteils 33 wird vorzugsweise auf geeignete Urformverfahren, insbesondere geeignete additive Fertigungsverfahren, wie beispielsweise selektives Laserschmelzen, 3D-Drucken oder andere pulvermetallurgische Verfahren, wie beispielsweise das MIM-Verfahren, zurückgegriffen, bei denen das gewünschte Metallteil 33 komplett einteilig in einheitlicher Struktur und Zusammensetzung gefertigt wird. In einem nächsten Fertigungsschritt wird das Metallteil 33 in einem geeigneten Werkzeug mit dem Werkstoffmantel 26 versehen. Dieser dringt insbesondere auch in die Spalte 27, 29 ein und dichtet diese nach außen hin ab. Vorzugsweise werden die Spalten 27, 29 dabei vollständig von dem Werkstoff ausgefüllt. Auch die Scharnieröffnung 19 kann ganz oder teilweise ausgefüllt werden. Beispielsweise kann mittels eines Formenkerns eine maßhaltige Lagerbohrung gebildet werden. Das Werkzeug zum Aufbringen des Werkstoffs kann eine Kunststoffspritzgussform, der Werkstoff kann ein Kunststoff sein.

Nach dem Aushärten des Kunststoffmantels 26 werden die Verbindungsstege 34 bis 39 entfernt. Diese können je nach Materialbeschaffenheit abgeschnitten, abgebrochen, abgerissen oder durch sonstige Maßnahmen, beispielsweise durch Laserbearbeitung, Stanzen, Schleifen oder Fräsen abgetrennt werden. Dies gilt sowohl bei Ausführungsformen des Verfahrens, bei denen die Verbindungsstege 34 bis 39 außerhalb des Werkstoffstoffmantels 26 liegen, als auch bei Verfahren, die auf den Verbindungsstegen 34 bis 39 Werkstoff hinterlassen.

Die erfindungsgemäße Branche 11 wird durch ein additives 3D-Fertigungsverfahren zur Herstellung eines Metallteils 33 erzeugt, das mindestens zwei Abschnitte umfasst, nämlich einen Trägerabschnitt 15 und einen Funktionsabschnitt 16, 17. Das Metallteil 33 ist ein einstückiges Teil, bei dem der Trägerabschnitt 15 und der Funktionsabschnitt 16, 17 durch entsprechende Verbindungsstege 34, 35 nahtlos miteinander verbunden sind. Nach Anbringen des Werkstoffs 26 werden die Verbindungsstege entfernt. Die so hergestellte Branche 11 ist maßgenau kompakt und weist hervorragende elektrische und thermische und mechanische Eigenschaften auf.

**Bezugszeichenliste**

| | |
|---|---|
| 10 | Werkzeugteil |
| 11,12 | Branchen |
| 13 | Schaft |
| 14 | Achse |
| 15 | Trägerabschnitt |
| 16,17 | Funktionsabschnitt |
| 18 | Finger |
| 19 | Scharnieröffnung |
| 20 | Durchgangsöffnung |
| 21 | Elektrodenplatte |
| 22 | Stelle |
| 23 | Haltestruktur |
| 24 | Fortsatz |
| 25 | Kopf |
| 26 | Werkstoff, Werkstoffmantel, Kunststoffmantel |
| 27 | Spalt |
| 28 | Hebel |
| 29 | Spalt |
| 30 | Fortsatz |
| 31 | Fortsatz |
| 32 | Ausnehmung |
| 33 | Metallteil |
| 34,35 | Verbindungsstege |
| 36-39 | Verbindungsstege |

## Patentansprüche

1. Verfahren zur Herstellung einer Branche (11) für ein elektrochirurgisches Instrument, mit folgenden Schritten:
Bereitstellen eines Metallteils (33), das einen Trägerabschnitt (15) und mindestens einen Funktionsabschnitt (16, 17) aufweist, die über mindestens einen Verbindungssteg (34 - 39) miteinander einteilig verbunden sind, Umspritzen des Metallteils (33) mit einem Werkstoff 26 aus Kunststoff,
- Entfernen des Verbindungsstegs (34 -
39), wobei
das Metallteil (33) durch selektives Laserschmelzen von Metallpulver bereitgestellt wird.

2. Verfahren nach Anspruch 1, **dadurch gekennzeichnet,**
**dass** der Funktionsabschnitt (16, 17) eine Elektrodenplatte (21) oder ein Hebel (28) ist.

3. Verfahren nach einem der vorstehenden Ansprüche,
**dadurch gekennzeichnet, dass** das Umspritzen unter Freilassung wenigstens eines Oberflächenbereichs des Funktionsabschnitts (16, 17) erfolgt.

4. Verfahren nach Anspruch 3, **dadurch**
**gekennzeichnet, dass** der Trägerabschnitt (15) und
der Funktionsabschnitt (16, 17) miteinander einen Spalt (27, 29) bilden, wobei an dem
Funktionsabschnitt (16, 17) mindestens eine auf der Seite des Trägerabschnitts (15) angeordnete Haltestruktur (23) ausgebildet wird, die den Trägerabschnitt (15) nicht berührt.

5. Elektrochirurgisches Instrument mit mindestens einer Branche (11), die:
einen Trägerabschnitt (15) und
mindestens einen Funktionsabschnitt (16, 17) aufweist, wobei zwischen dem Funktionsabschnitt (16) und dem Trägerabschnitt (15) ein Spalt (27) ausgebildet ist,
wobei der Funktionsabschnitt (16, 17) mit einer Haltestruktur (23) versehen ist, die in Form eines oder mehrerer Fortsätze (24) ausgebildet ist,
wobei sich die Fortsätze (24) von der dem Trägerabschnitt (15) zugewandten Seite des Funktionsabschnitts (16) weg in Richtung auf den Trägerabschnitt (15) hin in den Spalt (27) hinein erstrecken und den Trägerabschnitt (15) nicht berühren,
und wobei die Fortsätze (24) kontinuierlich oder an einer oder mehreren Stellen in zunehmendem Abstand zu dem Funktionsabschnitt (16) einen vergrößerten Querschnitt aufweisen, wodurch der Fortsatz (24) einen Kopf (25) ausbildet
wobei der Trägerabschnitt (15) und der Funktionsabschnitt (16, 17) aus Metall gleicher Zusammensetzung und Struktur ausgebildet und über einen Werkstoff (26), nämlich Kunststoff, miteinander fest verbunden sind, der den Spalt (27) ausfüllt.

6. Instrument nach Anspruch 5, **dadurch gekennzeichnet, dass** der Funktionsabschnitt (16, 17) eine Elektrodenplatte (21) oder ein Hebel (28) ist.

7. Instrument nach einem der Ansprüche 5 oder 6, **dadurch gekennzeichnet, dass** der Trägerabschnitt (15)und der Funktionsabschnitt (16, 17) in einem additiven Fertigungsverfahren hergestellt sind.

8. Instrument nach einem der Ansprüche 5 bis 7, **dadurch gekennzeichnet, dass** der Trägerabschnitt (15) und der Funktionsabschnitt (16, 17) in einem pulvermetallurgischen Verfahren hergestellt sind.

## Claims

1. Method for producing a branch (11) for an electrosurgical instrument, with the following steps:
provision of a metal part (33) having a carrier portion (15) and at least one function portion (16, 17) which are connected together integrally via at least one connecting web (34 - 39),
over-moulding of the metal part (33) with a plastic material,
removal of the connecting web (34 - 39),
wherein the metal part (33) is produced by selective laser melting of metal powder.

2. Method according to claim 1, **characterised in that** the function portion (16, 17) is an electrode plate (21) or a lever (28).

3. Method according to any of the preceding claims, **characterised in that** the over-moulding takes place leaving at least one surface region of the function portion (26, 27) clear.

4. Method according to claim 3, **characterised in that** the carrier portion (15) and the function portion (16, 17) together form a gap (27, 29), wherein at least one holding structure (23) is formed on the function portion (16, 17) and is arranged on the side of the carrier portion (15) without touching the carrier portion (15).

5. Electrosurgical instrument with at least one branch (11) having:
a carrier portion (15) and
at least one function portion (16, 17),
wherein a gap (27) is formed between the function portion (16) and the carrier portion (15),
wherein the function portion (16, 17) is provided with a holding structure (23) configured in the form of one or more extensions (24),
wherein the extensions (24) extend away from the side of the function portion (16) facing the carrier portion (15) into the gap (27) in the direction towards the carrier portion (15) and do not touch the carrier portion (15), and
wherein the extensions (24) have a cross-section which is enlarged continuously or at one or more locations with increasing distance from the function portion (16),
whereby the extension (24) forms a head (25),
wherein the carrier portion (15) and the function portion (16, 17) are made of metal of the same composition and structure and are fixedly connected together via a material (26), namely plastic, which fills the gap (27).

6. Instrument according to claim 5, **characterised in that** the function portion (16, 17) is an electrode plate (21) or a lever (28).

7. Instrument according to one of claims 5 or 6, **characterised in that** the carrier portion (15) and the function portion (16, 17) are produced in an additive production process.

8. Instrument according to one of claims 5 to 7, **characterised in that** the carrier portion (15) and the function portion (16, 17) are produced in a powder-metallurgical process.

## Revendications

1. Procédé de fabrication d'un mors (11) destiné à un instrument électrochirurgical, comprenant les étapes suivantes :
préparation d'une pièce en métal (33) qui présente une partie de support (15) et au moins une partie fonctionnelle (16, 17) qui sont reliées d'un seul tenant entre elles par au moins un élément de liaison (34 - 39),
enrobage de la pièce en métal (33) par injection avec un matériau constitué d'une matière plastique,
retrait de l'élément de liaison (34 - 39),
sachant que la pièce en métal (33) est préparée par fusion laser sélective de poudre métallique.

2. Procédé selon la revendication 1, **caractérisé en ce que** la partie fonctionnelle (16, 17) est une plaque d'électrode (21) ou un levier (28).

3. Procédé selon l'une des revendications précédentes, **caractérisé en ce que** l'enrobage par injection est réalisé en laissant dégagée au moins une zone de surface de la partie fonctionnelle (16, 17).

4. Procédé selon la revendication 3, **caractérisé en ce que** la partie de support (15) et la partie fonctionnelle (16, 17) forment entre elles une fente (27, 29), sachant que sur la partie fonctionnelle (16, 17), il est prévu au moins une structure de maintien (23) qui est disposée du côté de la partie de support (15) et qui n'est pas en contact avec la partie de support (15).

5. Instrument électrochirurgical comprenant au moins un mors (11) qui présente une partie de support (15) et au moins une partie fonctionnelle (16, 17),
une fente (27) étant formée entre la partie fonctionnelle (16, 17) et la partie de support (15),
la partie fonctionnelle (16, 17) étant dotée d'une structure de maintien (23) qui est réalisée sous la forme d'une ou plusieurs saillies (24),
les saillies (24) s'étendant à partir du côté de la partie fonctionnelle (16, 17) qui est tourné vers la partie de support (15), en direction de la partie de support (15), en pénétrant dans la fente (27), et ne sont pas en contact avec la partie de support (15), et
les saillies (24) présentant une section transversale qui augmente de façon continue ou à un ou plusieurs emplacements, à mesure que la distance par rapport à la partie fonctionnelle (16) augmente, ce qui a pour effet que la saillie (24) présente une tête (25),
la partie de support (15) et la partie fonctionnelle (16, 17) étant réalisées à partir d'un métal de même composition et de même structure et étant liées solidement l'une à l'autre par l'intermédiaire d'un matériau (26), à savoir une matière plastique, qui remplit la fente (27).

6. Instrument selon la revendication 5, **caractérisé en ce que** la partie fonctionnelle (16, 17) est une plaque d'électrode (21) ou un levier (28).

7. Instrument selon l'une des revendications 5 ou 6, **caractérisé en ce que** la partie de support (15) et la partie fonctionnelle (16, 17) sont réalisées au cours d'un procédé de fabrication additive.

8. Instrument selon l'une des revendications 5 à 7, **caractérisé en ce que** la partie de support (15) et la partie fonctionnelle (16, 17) sont réalisées au cours d'un procédé de métallurgie des poudres.
